Europäisches Patentamt

European Patent Office

Office européen des brevets

(11) Veröffentlichungsnummer: **0 255 640**
**B1**

## (12) EUROPÄISCHE PATENTSCHRIFT

(45) Veröffentlichungstag der Patentschrift:
07.06.89

(21) Anmeldenummer: 87110452.7

(22) Anmeldetag: 18.07.87

(51) Int. Cl.⁴: **B01J 27/28, C07C 51/377,
C07C 57/04**

---

(54) Regenerativ-katalytische Oxidehydrierung von Isobuttersäure oder deren Ester.

---

(30) Priorität: 02.08.86 DE 3626255

(43) Veröffentlichungstag der Anmeldung:
10.02.88 Patentblatt 88/6

(45) Bekanntmachung des Hinweises auf die Patenterteilung:
07.06.89 Patentblatt 89/23

(84) Benannte Vertragsstaaten:
DE ES FR GB IT NL

(56) Entgegenhaltungen:

(73) Patentinhaber: Röhm GmbH, Kirschenallee
Postfach 4242, D-6100 Darmstadt 1(DE)

(72) Erfinder: Gruber, Wilhelm, DR.,
Peter-Behrens-Strasse 34, D-6100 Darmstadt(DE)
Erfinder: Langerbeins, Klaus, Dr., Bahnstrasse 31-33,
D-6070 Langen(DE)
Erfinder: Ruppert, Wolfgang, Dr. Ing., Weedring 9,
D-6104 Seeheim-Jugenheim(DE)

---

ACTORUM AG

## Beschreibung

Gebiet der Erfindung

Die Erfindung betrifft die Regenerierung von bei der Oxidehydrierung von Isobuttersäure oder ihren Estern zu Methacrylsäure oder ihren Estern gebrauchten Katalysatoren, die als wesentliche Elemente Molybdän, Phosphor und Vanadium in oxidischem Verband enthalten und aus Heteropolysäuren des Molybdäns hergestellt sind.

Stand der Technik

Die katalytische Oxidehydrierung von Isobuttersäure oder ihren Estern zu Methacrylsäure oder ihren Estern ist allgemeiner Stand der Technik. Neben Katalysatoren auf Eisenphosphat-Basis haben Katalysatoren aus Heteropolysäuren des Molybdäns, insbesondere solche, die noch Phosphor und Vanadium und gegebenenfalls weitere Elemente, insbesondere Metallionen, enthalten, wegen ihrer höheren Selektivität deutliches Interesse gewonnen. Aktive und selektive Katalysatoren aus Hetereopolysäuren des Molybdäns, z.B. $H_5PMo_{10}V_2O_{40}$, oder deren Metallderivaten, sind beispielsweise in der DE-A 27 22 375 oder in der EP-C 0 079 491 oder in der EP-B 0 113 084 beschrieben.

Diese Materialien schließen Phosphormolybdänsäure der Formel
$H_3PMo_{12}O_{40}$
Vanadiumderivate derselben mit der Formel
$H_{3+x}PMo_{12-x}V_xO_{40}$,
wobei x = 1, 2 oder 3 ist,
die Metallsalze dieser Säuren und Kombinationen der Säuren, der Salze und der Säuren und Salze ein, wie sie in der Patentanmeldung EP-A 0 255 639, welche Stand der Technik im Sinne von Artikel 54(3) darstellt, beschrieben sind.

Sie werden vorzugsweise mit einem inerten Träger verdünnt angewendet. Solche für die Oxidehydrierung von Isobuttersäure oder ihrer niederen Ester verwendeten Katalysatoren halten nich über längere Zeit ihre nach dem Einfahren erreichten Aktivitäts- und Selektivitätsmaxima, wie dies für technische Prozesse notwenig ist, sondern verlieren nach einigen Tagen oder Wochen ständig an Wirksamkeit.

Der Verlust an katalytischer Effektivität ist ein häufig auftretendes Problem der Katalyse, auch der heterogenen Katalyse in der Gasphase. In Anbetracht der vielen Faktoren, die die Aktivität und Selektivität von Katalysatoren bei den verschiedensten katalytischen Verfahren beeinflussen, sind mögliche und bekannte Regenerierungsmaßnahmen sehr vielfältig. (Ullmanns Encyclopädie der techn. Chemie, 4. Auflage, Band 13, Seiten 518, 544 ff). Kostengünstige Regenerierungen sind dabei solche, in denen der Katalysator im Reaktor verbleiben kann.

Nach der US-A 4 052 333 wird die Regenerierung eines bei der Ammonoxidation von Olefinen desaktivierten molybdänhaltigen Katalysators der Zusammensetzung $Mo_{11,1}$ $Bi_1Co_{4,5}Ni_{2,5}Fe_{3,0}K_{0,41}P_{0,19}O_n$ durch zweistündiges Erhitzen auf 550 Grad C und Überleiter einer Luft-Wasserdampf-Mischung durchgeführt. Die Regenerierungstemperatur von 550 Grad C liegt ca. 120 Grad C und damit wesentlich über der beschriebenen Arbeitstemperatur des Katalysators bei der Ammonoxidation von Propylen.

Aufgabe und Lösung

Es bestand die Aufgabe, die Regenerierung gebrauchter Isobuttersäure(ester)-Oxidehydrierungskatalysatoren auf Heteropolymolybdat-Basis durchzuführen und ein Verfahren zur Oxidehydrierung und Regenerierung des Katalysators ohne Stillegung des katalysator-haltigen Reaktors zu entwickeln.

Es wurde gefunden, daß bei Oxidehydrierung von Isobuttersäure(ester) partiell desaktivierte - Molybdän, Phosphor und Vanadium als wesentliche Elemente enthaltende Heteropolymolybdat -Katalysatoren wieder regeneriert, d.h. reaktiviert und reselektiert werden, wenn diese im Temperaturbereich von 200 bis 400 Grad C, insbesondere von 250 bis 380 Grad C, oxidierend, mit einem sauerstoffhaltigen Gas, vor allem mit Luft behandelt werden. Die Erfindung betrifft demnach ein Verfahren zur Regenerierung von Katalysatoren, die Molybdän, Phosphor und Vanadium in oxidischer Form, sowie gegebenenfalls weiter Elemente, insbesondere Metalle als Kationen, enthalten, bei der Oxidehydrierung von Isobuttersäure oder ihren niederen Estern zu Methacrylsäure oder ihren niederen Estern, das dadurch gekennzeichnet ist, daß ein aus Phosphormolybdänsäure und/oder ihrer Vanadiumderivate und/oder deren Salzen hergestellter Katalysator zur Regenerierung im Temperaturbereich von 200 - 400 Grad C mit einem sauerstoffhaltigen Gas oxidierend behandelt wird.

Gegenüber dem Stand der Technik ist überraschend, daß molybdänhaltige Katalysatoren bei Temperaturen unter 400 Grad C oxidierend regeneriert werden. Es hat sich sogar überraschenderweise gezeigt, daß bei der Oxidehydrierung von Isobuttersäure oder ihrer Ester verwendete Katalysatoren auf Heteropolymolybdatbasis, wie beispielsweise solche aus $H_5PMo_{10}V_2O_{40}$ oder ihrer Metallsalze, bei Temperaturen über 400 Grad C nicht regenerierbar sind, sondern noch stärker desaktiviert werden.

Die Erfindung ermöglicht, die Regenerierung gebrauchten Katalysators an die Oxidehydrierung, ohne Durchführung umständlicher technischer Maßnahmen, anzuschließen.

Weiter wurde gefunden, daß die Katalysatordesaktivierung bei der Isobuttersäure- bzw. Isobuttersäureester-Oxidehydrierung wesentlich verlangsamt wird, wenn der Katalysator nach einer Oxidehydrierungsphase im Reaktorsystem den erfindungsgemäßen Regenerierungsbedingungen ausgesetzt wird, wobei die Regenerierungsphase genenüber der Oxidehydrierungsphase zeitlich verschieden, insbesondere kürzer sein kann. Der katalytische Prozeß kann so kontinuierlich bzw. kontinuierlich-periodisch betrieben werden. Die Erfindung ermöglicht die Herstellung von Methacrylsäure oder ihrer niederen Ester, wie Methacrylsäuremethylester, durch katalytische Oxidehydrierung von Isobuttersäure oder ihrer niederen Ester, wie Isobuttersäuremethylester, infolge wesentlicher Erhöhung der Standzeit der Katalysatoren, und ohne notwendige Prozeßunterbrechungen durch Reaktorstillegungen, wirtschaftlicher und technologisch einfacher gegenüber dem bisherigen Stand der Technik durchzuführen.

Ausführung der Erfindung

Die oxidative Dehydrierung von Isobuttersäure oder ihrer Ester zu Methacrylsäure oder ihrer Ester an Katalysatoren, die Molybdän, Phosphor, Vanadium und gegebenenfalls weitere Elemente insbesondere Metalle, als einen oxidischen Verband, vorzugsweise mit einem inerten Träger, wie beispielsweise aus der EP-C 0 079 491 bekannt, dispergiert, enthalten, und der Katalysator so in verschiedenen Formen und Größen (s. dazu Ullmanns Encyklopädie der technischen Chemie, 4. Auflage, Band 13, Seiten 558 bis 565) angewendet werden kann, wird im Temperaturbereich von 250 bis etwa 400 Grad C in Gegenwart von sauerstoffhaltigen Gasen, wie Luft, mit gegebenenfalls weiteren Inertgaszusätzen, wie beispielsweise Stickstoff, Wasserdampf, $CO_2$ oder Recycling-Gas aus der Reaktionsprodukt-Isolierung ausgeführt. Das Einsatzgasgemisch enthält pro Mol Isobuttersäure oder ihrem Ester etwa 1 bis 4 Mol Sauerstoff, normalerweise als Luftzusatz. Vor allem durch das Molverhältnis von Sauerstoff und Isobuttersäure(ester) wird die Selektivität des gewünschten Reaktionsprodukts bestimmt. Mit steigendem Sauerstoffanteil nimmt im allgemeinen die Menge des Verfahrensprodukts ab, und Nebenprodukte, insbesondere auch $CO_2$ und CO steigen deutlich an. Bei den Arbeitsweisen, die Methacrylsäure oder ihre Ester als Verfahrensprodukte anstreben, herrschen dann praktisch immer reduzierende Bedingungen am Katalysator, wodurch es in der Katalysatormatrix laufend zu weiteren Desaktivierungen katalytisch aktiver Stellen kommt. In niederer Wertigkeit vorliegende, katalytisch notwendige Elemente werden unter diesen Arbeitsbedingungen immer seltener wieder aufoxidiert, wodurch das Potential des Katalysators laufend abfällt.

Deswegen ist es notwendig gebrauchten Katalysator immer wieder reduzierenden Bedingungen zu entziehen und ihn dann oxidierenden, katalytisch regenerativen Bedingungen auszusetzen. Die Regenerierung ist erfindungsgemäß dadurch zu erreichen, daß gebrauchter Oxidationskatalysator im Temperaturbereich von 200 bis 400 Grad C oxidierend mit einem sauerstoffhaltigen Gas behandelt wird. Das oxidierend wirkende Regenerierungsgas ist vorzugsweise frei an oxidierbaren Kohlenstoffverbindungen. Es kann aber auch, gegebenenfalls geringe Mengen solcher Verbindungen enthalten. Die im oxidativ-regenerierend wirkenden Gas enthaltene Sauerstoffmenge ist dann größer als diejenige Sauerstoffmenge, die zu einer vollständigen Verbrennung gegebenenfalls darin noch enthaltener Kohlenstoffverbindungen benötigt wird. Der regenerierte Katalysator wird anschließend wieder katalytisch eingesetzt.

Der Sauerstoffgehalt des Regenerierungsgases liegt im Bereich von 5 - 40 Vol-%, vorzugsweise bei 10 bis 21 Vol-%. Die Regenerierung wird wie die Oxidehydrierung bei Drücken von etwa 0,1 bis etwa 5 bar, insbesondere bei Drücken von 0,5 bis 2,5 bar durchgeführt. Dies kann nach verschiedenen Verfahrenstechniken realisiert werden. Eine beispielsweise zwischenzeitliche Regenerierung des Katalysators wird so durchgeführt, daß dieser im Festbettreaktor praktisch unter den Temperaturbedingungen der Oxidehydrierung nach Abschaltung des zu oxidehydrierenden Substrates eine gewisse Zeit strömendem oxidierendem Gas, z.B. Luft, ausgesetzt wird. Die gewünschte Temperatur zwischen 200 und 400 Grad C läßt sich durch Abschalten des Substrates, und zwar so, daß hohe Verbrennungsraten und damit Überhitzungen am Katalysator wegen damit verbundener Zerstörung des katalytischen Gefüges vermieden werden, und gegebenenfalls entsprechender Vorwärmung des sauerstoffhaltigen Regenerierungsgases, das auch not Wasserdampf enthalten kann, einstellen. Das strömende oxidierende Gas kann auch noch mit weiteren Inertgasen, insbesondere zu Anfang der Regenerierung, verdünnt sein. Bei der Prozeßführung können die Oxidehydrierungsphasen im Bereich von einigen Minuten bis zu mehreren Stunden liegen, und die Regenerierungszeit soll möglichst nur wenige Minuten betragen, wodurch beispielsweise im Festbettreaktor eine intermittierende, jedoch praktisch kontinuierliche Arbeitsweise der Oxidehydrierung gegeben ist.Dadurch wird über die ganze Laufzeit ein vollkontinuierlicher Betrieb mit konstanten Raum-Zeit-Ausbeuten erreicht.

## BEISPIELE

### Beispiel 1

Ein dampfförmiges Gemisch aus Isobuttersäure und Luft im Molverhältnis Isobuttersäure : Sauerstoff, 1 : 1,5, wird an einem Katalysator, der aus 70 Gew.-% $H_5PMo_{10}V_2O_{40}$ und 25 Gew.-% Kieselgur sowie 5 Gew.-% Aerosil besteht, in einem Kreislaufreaktor gemäß DE-OS 30 19 731 bei 320 Grad C und einer Verweilzeit von 0,6 sec zur Reaktion gebracht. Die Belastung des Katalysators beträgt dabei 2,5 kg Isobuttersäure pro kg katalytische Masse und Stunde. Die erfindungsgemäße Regenerierung des Katalysators wird nach 75, 100, 150, und 200 Stunden durchgeführt. Die Regenerierungsphase im Luftstrom (Abschalten der Isobuttersäureversorgung) dauert jeweils 1 Stunde und wird bei 350 Grad C durchgeführt.

Tabelle 1

| Betriebszeit (h) | Isobuttersäure Umsatz (%) | Methacrylsäure Selektivität (%) | Regenerierung |
|---|---|---|---|
| 1 | 51 | 71 | |
| 30 | 47 | 71 | |
| 75 | 43 | 69 | |
| 76 | — | — | 1 h bei 350 Grad C |
| 80 | 50 | 70 | |
| 100 | 46 | 66 | |
| 101 | — | — | 1 h bei 350 Grad C |
| 105 | 50 | 68 | |
| 130 | 45 | 66 | |
| 150 | 43 | 64 | |
| 151 | — | — | 1 h bei 350 Grad C |
| 155 | 49 | 68 | |
| 180 | 47 | 66 | |
| 200 | 44 | 64 | |
| 201 | — | — | 1 h bei 350 Grad C |
| 205 | 49 | 67 | |
| 240 | 45 | 63 | |

### Vergleichsbeispiel

Ein unter gleichen Oxidehydrierungsbedingungen wie Beispiel 1 gefahrener gleicher $H_5PMo_{10}V_2O_{40}$-Katalysator zeigt ohne die erfindungsgemäße Regenerierung einen deutlich stärkeren Umsatz- und Selektivitätsabfall.

Tabelle 2

| Betriebszeit (h) | Isobuttersäure Umsatz (%) | Methacrylsäure Selektivität (%) |
|---|---|---|
| 1 | 51 | 68 |
| 150 | 30 | 64 |
| 250 | 22 | 53 |
| 300 | 21 | 50 |

### Beispiel 2

Ein dampfförmiges Gemisch aus Isobuttersäure und Sauerstoff (als Luft) im Molverhältnis 1 : 1,5 wird an einem wie im Beispiel 1 beschriebenen $H_5PMo_{10}V_2O_{40}$-Katalysator bei 320 Grad C und einer Verweilzeit von 0,6 sec. umgesetzt. Die Belastung des Katalysators beträgt 2,5 kg Isobuttersäure pro kg katalytische Masse und Stunde.

Die Regenerierung erfolgt innerhalb der ersten 210 h jeweils alle 2 h für 5 min und danach jeweils alle 27 min für 3 min. Die Temperatur wird bei der Regenerierung bei 320 Grad C gehalten.

Tabelle 3

| Betriebszeit (h) | Isobuttersäure Umsatz /%) | Mathacrylsäure Selektivität (%) | Regenerierung |
|---|---|---|---|
| 1 | 51 | 71 | alle 2 h |
| 20 | 51 | 71 | 5 min |
| 60 | 50 | 70 | Regenierung |
| 120 | 47 | 69 | |
| 180 | 46 | 68 | |
| 210 | 45 | 68 | |
| 220 | 45 | 68 | alle 27 min |
| 260 | 45 | 68 | 3 min |
| 330 | 44 | 67 | Regenerierung |

Beispiel 3

Ein dampfförmiges Gemisch aus Isobuttersäure, $O_2$ (als Luft) und $H_2O$ im molaren Verhältnis 1 : 1,5 : 0,5 wird über einen 70 Gew.-% $Cu_{0,2}H_{3,6}PMo_{11}V_1O_{40}$ und 30 Gew.-% Kieselsäure (Kieselgur : Aerosil 5 : 1) haltigen Katalysator (Herstellung nach EP-B 0 113 084) in einem Kreislaufreaktor bei 320 Grad C und einer Verweilzeit von 0,6 sec. zur Reaktion gebracht. Die Belastung des Katalysators beträgt 2,5 kg Isobuttersäure pro kg katalytische Masse und Stunde. Die 3-minütige Regenerierung erfolgt im Luftstrom bei 320 Grad C alle 27 min.

Tabelle 4

| Betriebszeit (h) | Isobuttersäure Umsatz(%) | Methacrylsäure Selektivität (%) | Regenerierung |
|---|---|---|---|
| 1 | 60 | 75 | alle 27 min |
| 60 | 60 | 75 | 3 min |
| 120 | 59 | 75 | Regenerierung |
| 180 | 57 | 75 | |
| 240 | 56 | 75 | |
| 300 | 55 | 74 | |

Vergleichsbeispiel 2

Ein unter gleichen Oxidehydrierungsbedingungen wie Beispiel 3 gefahrener $Cu_{0,2}H_{3,6}PMo_{11}V_1O_{40}$-Katalysator zeigt ohne Regnerierung und ohne $H_2O$-Zusatz einen wesentlich größeren Umsatz- und Selektivitätsabfall.

Tabelle 5

| Betriebszeit (h) | Isobuttersäure Umsatz(%) | Methacrylsäure Selektivität (%) |
|---|---|---|
| 1 | 50 | 72 |
| 60 | 44 | 71 |
| 120 | 33 | 70 |
| 180 | 25 | 66 |
| 240 | 21 | 61 |
| 300 | 19 | 55 |

Herstellung der Katalysatoren für die Beispiele 4 bis 10

Zur Herstellung der Heteropolysäurekatalysatoren der Beispiele 4, 5, 9 und 10 werden äquilmolare Mengen der bekannten Heteropolysäuren $H_5PMO_{10}V_2O_{40}$ und $H_4PMO_{11}VO_{40}$ und die formelmäßig angegebene Metalldotierung in wäßrigem Medium, gegebenenfalls unter Erhitzen, zusammengemischt und dann mit der Kieselsäure-Verdünnung zum Katalysator präpariert.

Cr wird als $CrO_3$, Mn als $Mn(CO_3)2$, Ce als $Ce((NO_3)_4$ und Cs als $Cs_2CO_3$ eingesetzt.

Zur Herstellung der Katalysatoren der Beispiele 6, 7 und 8 werden die Heteropolysäuren $H_4PMo_{11}VO_{40}$ bzw. $H_5PMo_{10}V_2O_{40}$ mit der formelgemäß angegebenen Metalldotierung im wäßrigen Medium zusammengemischt und dann mit der Kieselsäure-Verdünnung zum Katalysator präpariert.

Cu wird als CuO, Ti als $Ti(O-i-Propyl)_4$, Fe und Bi als Nitrate in salpetersaurer Lösung und K als KOH eingesetzt.

Oxidehydrierung mit Regenerierung

Ein dampfförmiges Gemisch aus Isobuttersäure, $O_2$ (aus Luft) und $N_2$ im molaren Verhältnis 1 : 1,5 : 7,71 wird über mit 30 Gew.-% Kieselsäure (Kieselgur : Aerosil 200 wie 5 : 1) verdünnten Heteropolysäurekatalysatoren bei 340 Grad C und einer Verweilzeit von 1 sec zur Reaktion gebracht. Die Belastung des Katalysators beträgt dabei 1,25 kg Isobuttersäure pro kg katalytischer Masse und Stunde.

Die Regenerierungsbedingungen sind in der Tabelle angegeben.

Die Ergebnisse der Oxidehydrierung mit zwischenzeitlicher Regenerierung sind aus der Tabelle 6 ersichtlich.

## Tabelle 6

| Katalysator | | Reaktionsergebnisse | | | | | | Regenerierungs-bedingungen | | | |
|---|---|---|---|---|---|---|---|---|---|---|---|
| | | $U_0$ [%] | $S_0$ [%] | $U_1$ [%] | $S_1$ [%] | $U_2$ [%] | $S_2$ [%] | T [°C] | t [min] | $O_2$-haltiges Gas ($O_2$:$N_2$-Verhältnis) | P [ata] |
| Bsp. 4 | $Cr_{0,2}H_{3,3}PMo_{10,5}V_{1,5}O_{40}$ | 74,3 | 71,0 | 69,2 (70 h) | 71,0 | 74,0 | 71,0 | 300 | 30 | 15:85 | 1 |
| Bsp. 5 | $Mn_{0,2}H_{4,1}PMo_{10,5}V_{1,5}O_{40}$ | 82,8 | 71,4 | 74,9 (80 h) | 71,4 | 83,0 | 71,0 | 360 | 15 | 21:79 | 1,5 |
| Bsp. 6 | $Cu_{0,2}Ti_{0,01}Fe_{0,01}Bi_{0,01}H_{3,5}PMo_{11}V_1O_{40}$ | 86,5 | 75,6 | 79,1 (120 h) | 75,0 | 86,7 | 75,0 | 340 | 15 | 21:79 | 1 |
| Bsp. 7 | $Cu_{0,05}H_{4,9}PMo_{10}V_2O_{40}$ | 80,2 | 72,5 | 72,8 (70 h) | 73,3 | 79,8 | 73,0 | 320 | 20 | 30:70 | 2 |
| Bsp. 8 | $K_{0,1}H_{4,9}PMo_{10}V_2O_{40}$ | 79,0 | 73,1 | 74,4 (80 h) | 73,4 | 79,5 | 72,5 | 340 | 20 | 21:79 | 0,5 |
| Bsp. 9 | $Ce_{0,4}H_{3,3}PMo_{10,5}V_{1,5}O_{40}$ | 84,7 | 72,0 | 81,2 (95 h) | 71,6 | 83,8 | 71,0 | 340 | 20 | 21:79 | 1 |
| Bsp. 10 | $Cs_{0,2}H_{4,3}PMo_{10,5}V_{1,5}O_{40}$ | 81,7 | 72,4 | 77,8 (120 h) | 72,0 | 81,0 | 71,7 | 340 | 20 | 21:79 | 1 |

$U_0$, $S_0$ Reaktionsergebnisse zu Beginn der Oxidehydrierung
$U_1$, $S_1$ Reaktionsergebnisse vor Regenerierung, nach bei $U_1$ angegebenen Reaktionszeiten
$U_2$, $S_2$ Reaktionsergebnisse nach Regenerierung

## Patentansprüche

1. Verfahren zur Regenerierung von Katalysatoren, die Molybdän, Phosphor und Vanadium in oxidischer Form, sowie gegebenenfalls weitere Elemente, insbesondere Metalle als Kationen, enthalten, bei der Oxidehydrierung von Isobuttersäure oder ihren niederen Estern zu Methacrylsäure oder ihren niederen Ester, dadurch gekennzeichnet,
daß ein aus Phosphormolybdänsäure und/oder ihrer Vanadiumderivate und/oder deren Salzen hergestellter Katalysator zur Regenerierung im Temperaturbereich von 200 bis 400 Grad C mit einem sauerstoffhaltigen Gas oxidierend behandelt wird.

2. Verfahren nach Anspruch 1, dadurch gekennzeichnet, daß das oxidierend wirkende Gas 5 bis 40 Vol-% Sauerstoff enthält.

3. Verfahren nach den Ansprüchen 1 und 2, dadurch gekennzeichnet,daß das oxidierend wirkende Gas 10 bis 21 Vol-% Sauerstoff enthält.

4. Verfahren nach den Ansprüchen 1 bis 3, dadurch gekennzeichnet, daß das Verfahren bei Drücken von 0,1 bis 5 bar ausgeführt wird.

5. Verfahren nach den Ansprüchen 1 bis 4, dadurch gekennzeichnet, daß das Verfahren intermittierend mit Oxidehydrierungsphasen im Festbettreaktor durchgeführt wird.

## Claims

1. Process for the regeneration of catalysts which contain molybdenum, phosphorus and vanadium in the form of oxide, and optionally further elements, in particular metals as cations, in the oxydehydration of isobutyric acid or its lower esters to give methacrylic acid or its lower esters, characterised in that a catalyst prepared from phosphomolybdic acid and/or its vanadium derivatives and/or its salts is oxidised with an oxygen-containing gas for regeneration in the temperature range from 200 to 400 degrees C.

2. Process according to claim 1, characterised in that the oxidising gas contains 5 to 40 volume % oxygen.

3. Process according to claims 1 and 2, characterised in that the oxidising gas contains 10 to 21 volume % oxygen.

4. Process according to claims 1 to 3, characterised in that the process is carried out at pressures of 0.1 to 5 bar.

5. Process according to claims 1 to 4, characterised in that the process is carried out in the solid bed reactor intermittently with oxydehydration phases.

## Revendications

1. Procédé pour la régénération de catalyseurs contenant du molybdène, du phosphore et du vanadium sous forme oxydée, et éventuellement d'autres éléments, en particulier des métaux sous forme de cations, lors de l'oxydéshydrogénation de l'acide isobutyrique ou de ses esters inférieurs pour donner de l'acide méthacrylique ou ses esters inférieurs, caractérisé en ce qu'un catalyseur, préparé à partir d'acide phosphomolybdique et/ou de ses dérivés du vanadium et/ou de ses sels, subit, pour être régénéré, un traitement d'oxydation à l'aide d'un gaz contenant de l'oxygène, sur l'intervalle de températures de 200 à 400°C.

2. Procédé selon la revendication 1, caractérisé en ce que le gaz à effet oxydant contient de 5 à 40% en volume d'oxygène.

3. Procédé selon les revendications 1 et 2, caractérisé en ce que le gaz à effet oxydant contient de 10 à 21% en volume d'oxygène.

4. Procédé selon les revendications 1 à 3, caractérisé en ce que le procédé est mis en œuvre à des pressions de 0,1 à 5 bar.

5. Procédé selon les revendications 1 à 4, caractérisé en ce qu'il est mis en œuvre d'une manière intermittente avec des phases d'oxydéshydrogénation dans un réacteur à lit fixe.